# EUROPEAN PATENT APPLICATION

(11) **EP 3 284 468 A1**
(43) Date of publication of application: **21.02.2018**
(21) Application number: 15889247.1
(22) Date of filing: 17.11.2015
(51) Int. Cl.: A61K 31/593, A61K 9/06, A61K 31/573, A61K 47/06, A61K 47/14, A61P 17/00, A61P 17/06, A61P 43/00

(54) **PHARMACEUTICAL COMPOSITION FOR SKIN**

(30) Priority: 15.04.2015 JP 2015083292
(71) Applicant: Maruho Co., Ltd., Osaka 531-0071 (JP)
(72) Inventor: EMI, Hidetoshi, Kyoto-shi Kyoto 600-8815 (JP); FUJII, Masahiro, Kyoto-shi Kyoto 600-8815 (JP)
(74) Representative: Pfenning, Meinig & Partner mbB
(86) International application number: PCT/JP2015/082186
(87) International publication number: WO 2016/166913

(57) **Abstract**

Disclosed herein is a pharmaceutical composition for skin that is a preparation containing a vitamin D₃ compound and a corticosteroid as active ingredients, that is excellent in the stability of each of the active ingredients, and that has appropriate transdermal absorbability of the active ingredients. The composition is a non-aqueous composition for skin comprising: (a) maxacalcitol; (b) at least one corticosteroid selected from the group consisting of betamethasone and esters thereof; (c) at least one low-polarity ester oil having an IOB value of 0.07 or more but less than 0.20; and (d) at least one non-polar liquid solvent.

## Description

### TECHNICAL FIELD

The present invention relates to a pharmaceutical composition for skin useful for treatment of inflammatory skin disorders (especially, psoriasis). More specifically, the present invention relates to a pharmaceutical composition for skin application comprising an active vitamin D₃ compound and a corticosteroid.

### BACKGROUND ART

Psoriasis is inflammatory keratosis characterized by abnormal growth of epidermal cells, dyskeratosis, inflammatory cell infiltration, and proliferation of blood vessels. Further, psoriasis is a refractory skin disorder that progresses with repeated remissions and exacerbations, and is typically associated with symptoms that silver-white scales adhere to slightly-raised red eruptions on the skin and then flake off like scurf. Psoriasis is basically treated by external application of a topical formulation, and its main therapy is combination therapy of a vitamin D₃ preparation having the effect of suppressing the growth of epidermal cells and the effect of inducing differentiation of epidermal cells and a corticosteroid preparation having anti-inflammatory effect.

It is known that a vitamin D₃ compound and a corticosteroid are different in chemical properties. For example, there is a difference in stable pH region between them. More specifically, a vitamin D₃ compound is stable at a weakly alkaline pH, but a steroid is stable at a weakly acidic pH. Therefore, if both of them are contained in one preparation, there is a problem that the quality of one or both of them is reduced.

For this reason, it is believed that an external preparation previously containing both of them is difficult to produce. Therefore, in a pharmacy or the like, a method has been used in which an external preparation containing a vitamin D₃ compound and an external preparation containing corticosteroid are mixed when needed and the resulting mixture is supplied to a patient. However, when two external preparations are mixed, there is a problem that adequate drug efficacy cannot be expected because a reduction in quality starts when they are mixed.

On the other hand, a vitamin D₃ preparation and a corticosteroid preparation may be prescribed for a patient as separate preparations, in which case the patient is instructed to apply one of the preparations and then apply the other preparation at some interval. However, this method in which two preparations are applied at different times is likely to cause a patient to forget about applying the other preparation, and therefore there is a fear that compliance is reduced. Particularly, since psoriasis is a chronic disorder, an external preparation needs to be used for a long time. For this reason, there has been demand for a preparation capable of applying both the active ingredients at one time.

Patent Document 1 discloses, as a combination preparation containing both a vitamin D₃ compound and a corticosteroid, a pharmaceutical composition for skin comprising at least one vitamin D or vitamin D derivative and at least one corticosteroid. More specifically, Example 1 in Patent Document 1 discloses a composition comprising betamethasone dipropionate as a corticosteroid, calcipotriol as a vitamin D derivative, liquid paraffin, polyoxypropylene-15-stearyl ether, α-tocopherol, and white petrolatum. Further, according to Patent Document 1, both the active ingredients contained in the above composition are very stable even when the composition is stored at 25°C for 3 months or at 40°C for 1 month/3 months.

Further, Patent Document 2 discloses, as a combination preparation containing both a vitamin D₃ compound and a corticosteroid, a storage stable ointment suitable for treating psoriasis comprising:
(a) a vitamin D compound;
(b) a corticosteroid; and
(c) an N,N-di(C₁-C₈)alkylamino substituted, (C₄-C₁₈) alkyl (C₂-C₁₈) carboxylic ester, or a (C₁-C₄)-alkyl (C₈-C₂₂) carboxylic ester in a petrolatum carrier, and optionally containing mineral oil, tocopherol, or both mineral oil and tocopherol.

Example in Patent Document 2 discloses that the ointment according to Patent Document 2 has storage stability comparable to that of TACLONEX (trademark) Ointment as a commercial product corresponding to the pharmaceutical composition according to Patent Document 1. Further, Patent Document 2 discloses that when the ointment according to Patent Document 2 is used, skin penetration of a vitamin D₃ compound (calcipotriene) is surprisingly enhanced as compared to TACLONEX (trademark), but skin penetration of a corticosteroid (betamethasone dipropionate) is reduced.

### PRIOR ART DOCUMENTS

### PATENT DOCUMENTS

Patent Document 1: JP-T-2002-542293
Patent Document 2: JP-T-2012-504607

### SUMMARY OF THE INVENTION

### PROBLEMS TO BE SOLVED BY THE INVENTION

As described above, Patent Documents 1 and 2 disclose compositions for skin containing both a vitamin D₃ compound and a corticosteroid. However, there has still been demand for a composition for skin that is capable of keeping a vitamin D₃ compound and a corticosteroid stable and that has appropriate transdermal absorbability of the active ingredients.

It is therefore an object of the present invention to provide a composition for skin that is a preparation containing both a vitamin D₃ compound and a corticosteroid, that is excellent in the stability of the active ingredients, and that has appropriate transdermal absorbability of the active ingredients.

### MEANS FOR SOLVING THE PROBLEMS

The composition for skin according to the present invention that can achieve the above object is as follows.

A non-aqueous composition for skin, comprising:
(a) maxacalcitol;
(b) at least one corticosteroid selected from the group consisting of betamethasone and esters thereof:
(c) at least one low-polarity ester oil having an IOB value of 0.07 or more but less than 0.20; and
(d) at least one non-polar liquid solvent.

The composition for skin according to the present invention contains the low-polarity ester oil (c) and the non-polar liquid solvent (d) and thereby makes it possible to keep the two active ingredients (a) and (b) stable in the composition.

Further, the transdermal absorbability of each of the active ingredients (a) and (b) contained in the composition is comparable to that of each product containing either one of the active ingredients that has been used in clinical sites for a long time and has established efficacy and safety. Therefore, the composition according to the present invention also has adequate safety in addition to adequate efficacy.

It is preferred that in the composition, a content of the low-polarity ester oil (c) is 1.5 to 4 wt%, a content of the non-polar liquid solvent (d) is 2 to 15 wt%, and the content of (d) is higher than the content of (c). It is to be noted that in this description, the term "content" refers to the ratio of the weight of each component to the total weight of the composition according to the present invention.

It is preferred that the low-polarity ester oil (c) is selected from the group consisting of octyldodecyl myristate, isostearyl palmitate, hexyldecyl isostearate, oleyl oleate, isocetyl myristate, stearyl stearate, decyl oleate, ethylhexyl stearate, cetyl caprate, octyl palmitate, cetyl 2- ethylhexanoate, isopropyl isostearate, hexyl laurate, isopropyl palmitate, isopropyl linoleate, and isopropyl myristate.

It is particularly preferred that the low-polarity ester oil (c) is selected from the group consisting of octyldodecyl myristate, isostearyl palmitate, hexyldecyl isostearate, oleyl oleate, isocetyl myristate, and stearyl stearate.

Further, preferable examples of the non-polar liquid solvent (d) include liquid paraffin and squalane.

It is preferred that the composition according to the present invention further comprises 80 wt% or more of a hydrocarbon oil that is solid or semi-solid at ordinary temperature (25°C).

Further, it is particularly preferred that the hydrocarbon oil is petrolatum.

It is preferred that in the composition according to the present invention,
the component (b) is betamethasone butyrate propionate,
the component (c) is selected from octyldodecyl myristate and oleyl oleate, the content thereof is 1.5 to 4 wt%,
the component (d) is selected from liquid paraffin and squalane, the content thereof is 2 to 15 wt%,
80 wt% or more of petrolatum is contained, and
a weight ratio between the components (c) and (d) is 1 : 1.3 to 4.5.

Further, it is particularly preferred that in the composition according to the present invention,
the component (b) is betamethasone butyrate propionate,
the component (c) is 1.5 to 2.5 wt% of octyldodecyl myristate,
the component (d) is 2.5 to 5 wt% of liquid paraffin,
90 wt% or more of petrolatum is contained, and
a weight ratio between the components (c) and (d) is 1 : 1.3 to 2.

### EFFECT OF THE INVENTION

The composition for skin according to the present invention contains a low-polarity ester oil and a non-polar liquid solvent in combination, and thereby makes it possible to keep both a vitamin D₃ compound (maxacalcitol) and a corticosteroid (betamethasone or an ester thereof) stable and to achieve appropriate transdermal absorbability of each of the components.

### BRIEF DESCRIPTION OF THE DRAWINGS

Fig. 1 is a graph showing the transdermal absorbability of maxacalcitol (OCT).
Fig. 2 is a graph showing the transdermal absorbability of betamethasone butyrate propionate (BBP).
Fig. 3 is a graph showing temporal changes in PSI total score when a composition according to the present invention, an OCT-containing preparation, and a BBP-containing preparation were each administered to patients with psoriasis vulgaris.

### MODE FOR CARRYING OUT THE INVENTION

Maxacalcitol (a) used in the present invention is an active vitamin D₃ derivative, and has the following structural formula and chemical name. Maxacalcitol is known to have lower stability than calcipotriol used in Example in Patent Document 1, but the composition according to the present invention is excellent in stability of maxacalcitol.

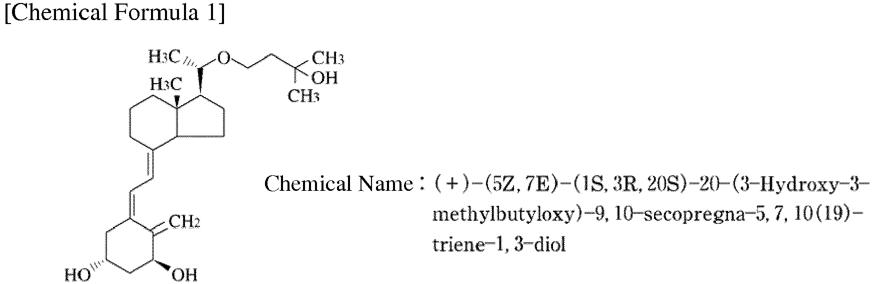

The content of the maxacalcitol (a) in the composition according to the present invention is preferably 0.0005 to 0.02 wt%, more preferably 0.001 to 0.01 wt%, and particularly preferably 0.002 to 0.005 wt%.

The corticosteroid (b) used in the present invention is betamethasone or a pharmaceutically-acceptable ester thereof. The content of the corticosteroid (b) in the composition according to the present invention is preferably 0.01 to 0.5 wt%, more preferably 0.02 to 0.25 wt%, and particularly preferably 0.03 to 0.1 wt%. The composition according to the present invention may contain one corticosteroid (b) or two or more corticosteroids (b), but more preferably contains one corticosteroid (b).

Examples of the ester of betamethasone include betamethasone butyrate propionate, betamethasone valerate, betamethasone dipropionate, and betamethasone phosphate. Particularly preferably, the corticosteroid is betamethasone butyrate propionate having the following structural formula and chemical name.

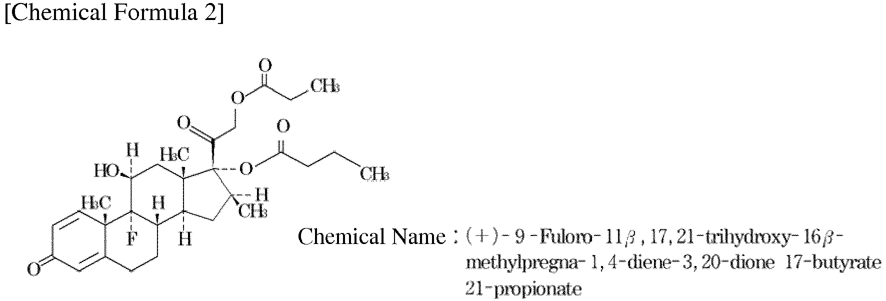

The low-polarity ester oil (c) used in the present invention is liquid at ordinary temperature (25°C), and has an IOB (Inorganic/Organic Balance) value of 0.07 or more but less than 0.20 (more preferably 0.16 or less, particularly preferably 0.10 or less).

The IOB value is an indicator for the degree of polarity of an organic compound, and a lower IOB value indicates lower polarity. The IOB value is represented by the formula IOB value = inorganic value/organic value, and the calculation method thereof is described in "Organic Conceptual Diagram-Foundation and Application" (written by Yoshio Koda, published by SANKYO SHUPPAN Co., Ltd. in 1984) etc.

An aliphatic carboxylic acid and an aliphatic alcohol constituting the low-polarity ester oil (c) used in the present invention may be either linear or branched and may be either saturated or unsaturated. Examples of the low-polarity ester oil include fatty acid esters listed in the following table.

**[Table 1]**

| Name | Structural characteristics | IOB value | Name | Structural characteristics | IOB value |
|---|---|---|---|---|---|
| Octyldodecyl myristate | Carboxylic acid (C14 linear) | 0.09 | Cetyl caprate | Carboxylic acid (C10 linear) | 0.12 |
| | Alcohol (C20 branched) | | | Alcohol (C16 linear) | |
| Isostearyl palmitate | Carboxylic acid (C16 linear) | 0.09 | Octyl palmitate | Carboxylic acid (C16 linear) | 0.13 |
| | Alcohol (C18 branched) | | | Alcohol (C8 branched) | |
| Hexyldecyl isostearate | Carboxylic acid (C18 branched) | 0.09 | Cetyl 2-ehtylhexanoate | Carboxylic acid (C8 branched) | 0.13 |
| | Alcohol (C16 branched) | | | Alcohol (C16 linear) | |
| Oleyl oleate | Carboxylic acid (C18 linear) | 0.09 | Isopropyl isostearate | Carboxylic acid (C18 branched) | 0.15 |
| | Alcohol (C18 linear) | | | Alcohol (C3 branched) | |
| Isocetyl myristate | Carboxylic acid (C14 linear) | 0.10 | Hexyl laurate | Carboxylic acid (C12 linear) | 0.17 |
| | Alcohol (C16 branched) | | | Alcohol (C6 linear) | |
| Stearyl stearate | Carboxylic acid (C18 linear) | 0.08 | isopropyl palmitate | Carboxylic acid (C16 linear) | 0.16 |
| | Alcohol (C18 linear) | | | Alcohol (C3 branched) | |
| Decyl oleate | Carboxylic acid (C18 linear) | 0.11 | Isopropyl linoleate | Carboxylic acid (C18 linear) | 0.16 |
| | Alcohol (C10 linear) | | | Alcohol (C3 branched) | |
| Ethylhexyl stearate | Carboxylic acid (C18 linear) | 0.12 | Isopropyl myristate | Carboxylic acid (C14 linear) | 0.18 |
| | Alcohol (C8 branched) | | | Alcohol (C3 branched) | |

The low-polarity ester oil (c) used in the present invention is more preferably an ester oil having an IOB value of 0.10 or less. Examples of such an ester oil include ester oils derived from C₁₂ to C₂₀ (especially, C₁₄ to C₁₈) aliphatic carboxylic acids and C₁₄ to C₂₄ (especially, C₁₆ to C₂₀) aliphatic alcohols, especially octyldodecyl myristate, isostearyl palmitate, hexyldecyl isostearate, oleyl oleate, isocetyl myristate, and stearyl stearate.

The low-polarity ester oil (c) is particularly preferably octyldodecyl myristate or oleyl oleate.

The content of the low-polarity ester oil (c) is preferably 1.5 to 4 wt%, more preferably 1.5 to 3.5 wt%, and particularly preferably 2 to 3 wt%. Further, the composition according to the present invention may contain one low-polarity ester oil or two or more low-polarity ester oils, but preferably contains one low-polarity ester oil.

Examples of the non-polar liquid solvent (d) used in the present invention include liquid paraffin and squalane. The lower limit of the content of the non-polar liquid solvent is preferably 2 wt%, more preferably 2.5 wt%, and particularly preferably 3 wt%. Further, the upper limit of the content of the non-polar liquid solvent is preferably 15 wt%, more preferably 13 wt%, particularly preferably 12 wt%, even more preferably 5 wt%. These upper and lower limits can be arbitrarily combined, but the content of the non-polar liquid solvent is more preferably in the range of 2.5 to 13 wt% (especially, 5 wt% or less), particularly preferably 3 to 12 wt% (especially, 5 wt% or less). The composition according to the present invention may contain one non-polar liquid solvent (d) or two or more non-polar liquid solvents (d), but more preferably contains one non-polar liquid solvent (d).

In the composition for skin according to the present invention, the content of the non-polar liquid solvent (d) is preferably larger than that of the low-polarity ester oil (c). More specifically, the weight ratio between (c) and (d) is more preferably (c) : (d) = 1 : 1.3 to 4.5, particularly preferably 1 : 1.3 to 4, even more preferably 1 : 1.3 to 2, most preferably 1 : about 1.5 (1 : 1.4 to 1.6).

Further, the composition for skin according to the present invention preferably contains, as a base ingredient, a hydrocarbon oil that is solid or semi-solid at ordinary temperature (25°C). Examples of the hydrocarbon oil include petrolatum (especially, white petrolatum), paraffin, and gelled hydrocarbons. The hydrocarbon oil is particularly preferably petrolatum (especially, white petrolatum). The content of the hydrocarbon oil is preferably 80 to 96 wt%, more preferably 84 to 95.5 wt%, and particularly preferably 90 to 95 wt%.

The composition for skin according to the present invention is a non-aqueous composition, and therefore contains substantially no water. The phrase "contains substantially no water" means that water is not intentionally added in a production process. Therefore, the water content of the composition according to the present invention is usually less than 1 wt% (more preferably less than 0.5 wt%, particularly preferably 0 wt%).

A preferred example of a dosage form of the composition for skin according to the present invention is an ointment. The application frequency, dosage, etc. of the composition for skin according to the present invention may be appropriately adjusted depending on the concentrations of the active ingredients, the age and body weight of a patient, the area of skin having symptoms, the degree of symptoms, etc. Usually, the composition according to the present invention may be applied to the skin having symptoms in an appropriate amount (in an amount such that an affected area is thinly coated therewith) once a day so that the amount of maxacalcitol does not exceed 250 µg (corresponding to 10 g of a preparation containing 25 µg of maxacalcitol per gram) and the amount of the corticosteroid does not exceed 5 mg (corresponding to 10 g of a preparation containing 0.5 mg of the corticosteroid per gram).

A preferred example of the composition for skin according to the present invention is a non-aqueous composition containing the low-polarity ester oil (c) in an amount of 1.5 to 4 wt% (more preferably 1.5 to 3.5 wt%, particularly preferably 2 to 3 wt%) and the non-polar liquid solvent (d) in an amount of 2 to 15 wt% (more preferably 2.5 to 13 wt%, particularly preferably 3 to 12 wt%) and having a weight ratio between the (c) and the (d) of 1 : 1.3 to 4.5 (more preferably 1.5 to 4). The (c) is particularly preferably one having an IOB value of 0.1 or less.

A particularly preferred example of the composition for skin according to the present invention is a non-aqueous composition comprising:
(a) maxacalcitol;
(b) betamethasone butyrate propionate;
(c) 1.5 to 4 wt% (more preferably 1.5 to 3.5 wt%) of a low-polarity ester oil selected from octyldodecyl myristate and oleyl oleate;
(d) 2 to 15 wt% (more preferably 2.5 to 13 wt%) of a non-polar liquid solvent selected from liquid paraffin and squalane, and 80 wt% or more of petrolatum (preferably white petrolatum), wherein a weight ratio between the (c) and the (d) is 1 : 1.3 to 4.5 (more preferably 1 : 1.5 to 4).

An even more preferred example of the composition for skin according to the present invention is a non-aqueous composition comprising:
(a) maxacalcitol;
(b) betamethasone butyrate propionate;
(c) 1.5 to 2.5 wt% (more preferably 1.8 to 2.2 wt%) of octyldodecyl myristate;
(d) 2.5 to 5 wt% (more preferably 2.5 to 4 wt%) of liquid paraffin, and 90 wt% or more of petrolatum (preferably white petrolatum), wherein a weight ratio between the (c) and the (d) is 1 : 1.3 to 2 (more preferably 1 : 1.4 to 1.6).

The composition for skin according to the present invention may contain a dissolution aid for the active ingredients. Examples of the dissolution aid include anhydrous ethanol, methanol, isopropanol, and methyl ethyl ketone. The content of the dissolution aid may be usually 0.02 to 1 wt%.

Further, the composition for skin according to the present invention may contain other additives generally used for external applications for skin. Examples of such additives include antioxidants (e.g., ascorbic acid, dibutyl hydroxy toluene, and tocopherol acetate), stabilizers (e.g., edetate sodium and L-menthol), preservatives (e.g., p-hydroxybenzoate), and colorants (e.g., titanium oxide).

The total amount of the dissolution aid and other additives is preferably 5 wt% or less, more preferably 3 wt% or less, particularly preferably 2 wt% or less.

In the preceding paragraphs, the names of compounds of the essential and optional components that can be used in the composition according to the present invention have been described. It is to be noted that the composition according to the present invention includes compositions obtained by arbitrarily combining these compounds and compositions obtained by arbitrarily combining wt% and the weight ratio described about the essential and optional components. Further, when two or more numerical ranges are described, the upper limits of the respective numerical ranges and the lower limits of the respective numerical ranges may be arbitrarily combined.

Hereinbelow the present invention will be described in more detail with reference to Examples, but the present invention is not limited to Examples.

### [Example 1]

Compositions were prepared for the purpose of developing a composition for skin in which transdermal absorbability of each of a vitamin D₃ compound and a corticosteroid is comparable to that of each existing product containing either one of the active ingredients (hereinafter, referred to as a single-drug product). This is because these single-drug products have been used in clinical sites for a long time, and their efficacy and safety have been clarified. Therefore, when a composition containing the two active ingredients shows absorbability of each of the active ingredients comparable to that of each of the single-drug products, the efficacy of the composition can be expected to be higher than that of each of the single-drug products. Further, it has been clarified that in a non-clinical test, the two active ingredients do not toxically interact with each other. Therefore, it can be considered that when the composition shows absorbability of each of the two active ingredients comparable to that of each of the single-drug products, the same safety as each of the single-drug products can be ensured in clinical use. If the composition shows absorbability of each of the two active ingredients lower than that of each of the single-drug products, the same efficacy as each of the single-drug products cannot be ensured. On the other hand, if the composition shows absorbability of each of the two active ingredients higher than that of each of the single-drug products, its safety cannot be ensured.

Therefore, a preparation containing both a vitamin D₃ compound (maxacalcitol, hereinafter referred to as OCT) and a corticosteroid (betamethasone butyrate propionate, hereinafter, referred to as BBP) was designed to achieve transdermal absorbability of each of the active ingredients comparable to that when each approved product (Oxarol Ointment and Antebate Ointment, respectively) containing either one of the active ingredients was used. OCT is present in a dissolved state in Oxarol Ointment, and BBP is present in a dispersed state in Antebate Ointment. Therefore, an experiment was performed to find a solvent in which OCT can be dissolved and BBP can be dispersed, but it was difficult to achieve a desired composition using a single solvent. As a result of investigation, a desired composition was successfully obtained by using a non-polar (IOB = 0) liquid solvent and a low-polarity (0.07 ≤ IOB < 0.2) ester oil.

Examples (No. 1 and No. 2) of the composition according to the present invention are shown in Table 2. It is to be noted that a composition No. 3 is a comparative composition containing a medium-chain fatty acid triglyceride (medium-polarity ester oil having an IOB value higher than 0.2) instead of a low-polarity ester oil used in the present invention, and a composition No. 4 is a comparative composition containing no low-polarity ester oil. The dosage form of each of the compositions is an ointment. Whether OCT and BBP were dissolved or dispersed was determined by observation with a microscope.

### [Table 2]

**Table 2 (Numerical values for each component in the table are expressed in % by weight)**

| | Name of component | | No.1 | No.2 | No.3 | No.4 |
|---|---|---|---|---|---|---|
| Vitamin D₃ compound (a) | Maxacalcitol (OCT) | | 0.0025 | 0.0025 | 0.0025 | 0.0025 |
| Corticosteroid (b) | Betamethasone butyrate propionate (BBP) | | 0.05 | 0.05 | 0.05 | 0.05 |
| Low-polarity ester oil (c) | Octyldodecyl myristate (IOB: 0.09) | | 2 | 3 | - | - |
| Medium-polarity ester oil | Medium-chain fatty acid triglyceride (IOB > 0.2) | | - | - | 5 | - |
| Non-polar liquid solvent (d) | Liquid paraffin | | 3 | - | - | 15 |
| | Squalane | | - | 12 | - | - |
| Dissolution aid | Anhydrous ethanol | | 0.0475 | 0.0475 | 0.0475 | 0.0475 |
| Solid or semi-solid hydrocarbon oil | White petrolatum | | 94.9 | 84.9 | 94.9 | 84.9 |
| State of active ingredient present in composition | OCT | | Dissolved | Dissolved | Dissolved | Dissolved |
| | BBP | | Dispersed | Dispersed | Dissolved | Dispersed |
| Stability (40°C, 12 weeks) | Scale: 100 g | OCT | 100.6% | - | 103.0% | 95.3% |
| | | BBP | 100.5% | - | 100.3% | 100.3% |
| Stability (40°C, 12 weeks) | Scale: 200 g | OCT | 99.7% | 99.8% | - | - |
| | | BBP | 100.4% | 99.9% | - | - |
| Stability (40°C, 26 weeks) | Scale: 300 g | OCT | 97.9% | 98.2% | - | - |
| | | BBP | 99.0% | 98.7% | - | - |

Each of the compositions shown in Table 2 was prepared by the following procedure.

### [Preparation of Composition]

(Composition No. 1) OCT was dissolved in anhydrous ethanol, and then the resulting solution was added to and mixed with octyldodecyl myristate. Further, BBP was added to and mixed with liquid paraffin. These OCT mixture and BBP mixture were added to white petrolatum separately prepared by melting at 70°C, and the resulting mixture was cooled to room temperature with stirring.

(Composition No. 2) A composition No. 2 was produced in the same manner as the composition No. 1 except that the liquid paraffin was replaced with squalane.

(Composition No. 3) OCT was dissolved in anhydrous ethanol, and then the resulting solution and BBP were added to and mixed with a medium-chain fatty acid triglyceride. The resulting mixture was added to white petrolatum separately prepared by melting at 70°C, and the resulting mixture was cooled to room temperature with stirring.

(Composition No. 4) A composition No. 4 was produced in the same manner as the composition No. 3 except that the medium-chain fatty acid triglyceride was replaced with liquid paraffin.

The safety of each of the active ingredients (the residual ratio of each of the active ingredients after storage) shown in Table 2 was determined by the following test.

### [Stability Test of Active Ingredients (a) and (b) in Compositions]

The stability test of the active ingredients (a) and (b) in each of the compositions No.1 to No. 4 was performed under accelerated conditions. The amounts of the active ingredients (a) and (b) contained in each of the compositions were measured after the compositions were stored for a certain period of time in a constant temperature and humidity chamber set to 40°C and 75%RH.

The active ingredient (a) was measured in the following manner. A hexane solution (a hexane solution of 3-tert-butyl-4-hydroxyanisole), acetonitrile, and an internal standard solution were added to about 0.5 g of each sample, and the resulting mixture was shaken to fractionate an acetonitrile layer. The solvent was distilled away at room temperature under a reduced pressure, and then the resulting dried solid was dissolved by adding a mixed solution of hexane and 1-butanol (hexane : 1-butanol = 19:1) to obtain a sample solution. The amount of the active ingredient (a) contained in each sample solution was measured by high-performance liquid chromatography (column: TSKgel OH-120 (manufactured by Tosoh Corporation), detection wavelength: 265 nm, mobile phase: mixed solution of hexane : 1-butanol: acetic acid (100) = 19 : 1 : 1). The residual ratio (%) relative to the initial value of the active ingredient (a) was determined.

Further, the active ingredient (b) was measured in the following manner. Tetrahydrofuran, acetonitrile, and an internal standard solution were added to about 1 g of each of the compositions, and the resulting mixture was shaken. The resulting solution was filtered through a membrane filter to obtain a sample solution. The amount of the active ingredient (b) contained in the sample solution was measured by high-performance liquid chromatography (column: XBridge C18 (manufactured by Nihon Waters K.K.), detection wavelength: 240 nm, mobile phase: mixed solution of water : acetonitrile : tetrahydrofuran = 13 : 6 : 2). The residual ratio (%) relative to the initial value of the active ingredient (b) was determined.

As can be seen from Table 2, the residual ratio of each of the active ingredients contained in each of the compositions No. 1 and No. 2 according to the present invention was high even after storage at 40°C for 26 weeks (about 6 months). On the other hand, the comparative composition No. 4 containing no low-polarity ester oil was poor in the stability of OCT.

### [Example 2]

### [In Vivo Transdermal Absorption Test Using Hairless Mice]

The compositions No. 1 to No. 4 produced in Example 1 were subjected to a transdermal absorption test, and their transdermal absorptivities were compared to those of a commercial product containing OCT alone (Oxarol Ointment, OCT content: 0.0025 wt%) and a commercial product containing BBP alone (Antebate Ointment, BBP content: 0.05 wt%).

A plastic frame (4 cm²) was fixed to each hairless mouse under anesthesia. Then, 20 mg of a sample was transdermally administered by applying the sample to the skin within the frame, and the frame was fixed with an adhesive bandage. After a lapse of 1, 4, and 8 hours, the surface of the skin to which the sample had been administered was wiped with cotton soaked with 70% ethanol under anesthesia. Each of the hairless mice was sacrificed by exsanguination, and then the skin where the sample had been administered was collected. The collected skin was homogenized in acetonitrile, and then a liquid phase was collected. The liquid phase was dried and then dissolved in a dissolution solvent (acetonitrile) to obtain a sample solution. The sample solution was subjected to liquid chromatography/tandem mass spectrometry (LC/MS/MS) to calculate the concentrations of OCT and BBP in the skin.

The measurement conditions were as follows.

### [Table 3]

### Conditions of LC/MS/MS

### <Conditions of LC>

Analytical column: Capcellpak MG, Inner diameter: 2.0 mm, Length 50 mm, Particle diameter 3 µm (manufactured by Shiseido Company, Limited)
Mobile phase A: 2 mmol/L aqueous ammonium acetate solution,
Mobile phase B: Methanol
Gradient:

| Time (min) | A (%) | B (%) | Flow rate (mL/min) |
|---|---|---|---|
| 0.0 - 3.0 | 50 → 5 | 50 → 95 | 0.3 |
| 3.0 - 5.0 | 5 | 95 | 0.3 |
| 5.0 - 5.1 | 5 → 50 | 95 → 50 | 0.3 |
| 5.1 - 8.0 | 50 | 50 | 0.3 |

Analysis time: 8 min, Column temperature: 40°C, Sample temperature: 4°C,
Injection amount: 10 µL

### <Conditions of MS/MS>

Ionization method: Turbo ion spray, Positive ion mode
Target ion:

| Compound | Q1 (*m*/*z*) | Q3 (*m*/*z*) |
|---|---|---|
| OCT | 436.40 | 297.40 |
| BBP | 520.51 | 412.10 |
| Prednisolone (Internal standard) | 361.11 | 325.25 |

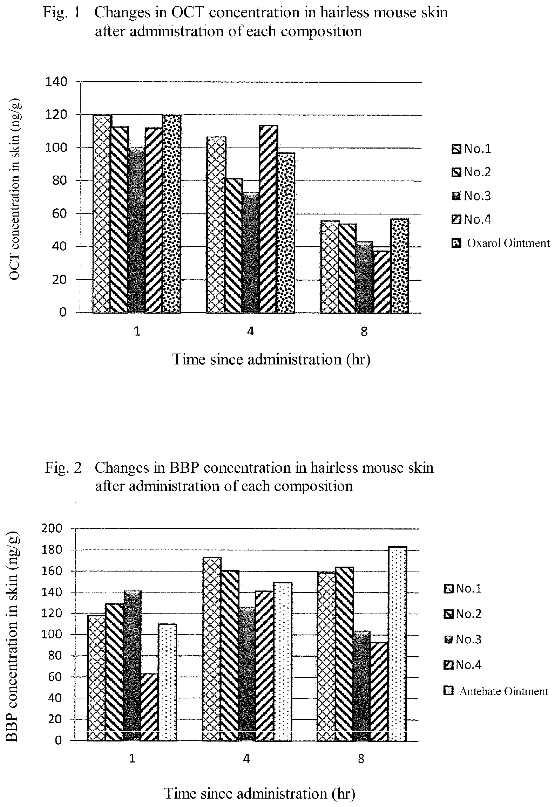

The results of the transdermal absorption test are shown in Table 4 and Figs. 1 and 2. Further, the compositions No. 1 to No. 4 were evaluated as to whether or not the transdermal absorbability of OCT and the transdermal absorbability of BBP were both comparable to that of Oxarol ointment and that of Antebate Ointment, respectively. More specifically, each composition was evaluated as "comparable", if the concentrations of OCT and BBP in the skin at each measurement time (1 hour, 4 hours, 8 hours) were in the range of ± 20% of the concentration of OCT in the skin when Oxarol Ointment was used, and in the range of ± 20% of the concentration of BBP in the skin when Antebate Ointment was used, respectively. The symbol "*" shown in (1) and (2) in Table 4 means that the measured value was not in the range of ± 20%. Further, in (3) in Table 4, the symbol "○" means that the transdermal absorbability of each of the active ingredients of the composition can be evaluated as comparable to that of each of the single-drug products, and the symbol "×" represents the composition not to be evaluated as "comparable".

### [Table 4]

**Table 4 Results of transdermal absorption test**

| (1) Concentration of OCT in skin (ng/g) | | | |
|---|---|---|---|
| | 1 hr | 4 hr | 8 hr |
| Composition No.1 | 119.7 | 106.7 | 55.86 |
| Composition No.2 | 112.5 | 81.21 | 54.12 |
| Composition No.3 | 100.6 | 73.31* | 43.43* |
| Composition No.4 | 111.9 | 113.6 | 37.34* |
| Oxarol Ointment | 119.6 | 97.00 | 57.22 |

| (2) Concentration of BBP in skin (ng/g) | | | |
|---|---|---|---|
| | 1 hr | 4 hr | 8 hr |
| Composition No.1 | 117.9 | 173.1 | 158.6 |
| Composition No.2 | 128.9 | 160.4 | 163.8 |
| Composition No.3 | 142.0* | 126.1 | 103.9* |
| Composition No.4 | 62.97* | 140.9 | 93.03* |
| Antebate Ointment | 109.8 | 149.6 | 183.4 |

| (3) Evaluation of transdermal absorbability | | | |
|---|---|---|---|
| Composition No.1 | ○ | | |
| Composition No.2 | ○ | | |
| Composition No.3 | × | | |
| Composition No.4 | × | | |

As shown in Table 4, the compositions No. 1 and No. 2 according to the present invention showed transdermal absorbability of each of the active ingredients comparable to that of each of the single-drug products. On the other hand, the comparative composition No. 3 containing a medium-polarity, medium-chain fatty acid triglyceride and the comparative composition No. 4 containing no low-polarity ester oil did not show transdermal absorbability comparable to that of each of the single-drug products.

### [Example 3]

Compositions (ointments) shown in Table 5 were prepared. Compositions No. 5 and No. 6 shown in Table 5 are compositions according to the present invention, and a composition No. 7 is a comparative composition using polyoxypropylene-15-stearyl ether (PPG-15 stearyl ether) used in Example in Patent Document 1 instead of a low-polarity ester oil.

The composition No. 5 was produced in the same manner as the composition No. 1, and the compositions No. 6 and No. 7 were produced in the same manner as the composition No. 1 except that octyldodecyl myristate was replaced with oleyl oleate or PPG-15 stearyl ether.

The stability (residual ratio after storage) of OCT and BBP of each of the compositions No. 5 to No. 7 was determined in the following manner.

### [Stability Test of Active Ingredients (a) and (b) in Compositions]

The amounts of the active ingredients (a) and (b) contained in each of the compositions were measured after the compositions were stored for 8 weeks in a constant temperature and humidity chamber set to 50°C.

The active ingredients (a) and (b) were measured in the following manner. A hexane solution (a hexane solution of 3-tert-butyl-4-hydroxyanisole), acetonitrile, and an internal standard solution were added to about 0.5 g of each sample, and the resulting mixture was shaken to fractionate an acetonitrile layer. The solvent was distilled away at room temperature under a reduced pressure, and then the resulting dried solid was dissolved by adding a mixed solution of hexane and 1-butanol (hexane : 1-butanol = 19:1) to obtain a sample solution. The amounts of the active ingredients (a) and (b) contained in each sample solution were measured by high-performance liquid chromatography (column: TSKgel OH-120 (manufactured by Tosoh Corporation), detection wavelength: 265 nm, mobile phase: mixed solution of hexane : 1-butanol: acetic acid (100) = 19 : 1 : 1). The residual ratio (%) relative to the initial value of each of the active ingredients (a) and (b) was determined.

### [Table 5]

**Table 5 (Numerical values for each component in the table are expressed in % by weight)**

| | Name of component | No.5 | No.6 | No.7 |
|---|---|---|---|---|
| Vitamin D₃ compound (a) | Maxacalcitol (OCT) | 0.0025 | 0.0025 | 0.0025 |
| Corticosteroid (b) | Betamethasone butyrate propionate (BBP) | 0.05 | 0.05 | 0.05 |
| Low-polarity ester oil (c) | Octyldodecyl myristate (IOB: 0.09) | 2 | - | - |
| | Oleyl oleate (IOB: 0.09) | - | 2 | - |
| Medium-polarity ether | PPG-15 stearyl ether (IOB: 0.36) | - | - | 2 |
| Non-polar liquid solvent (d) | Liquid paraffin | 3 | 3 | 3 |
| Dissolution aid | Anhydrous ethanol | 0.0475 | 0.0475 | 0.0475 |
| Preservative | Propyl parahydroxybenzoate | 0.03 | 0.03 | 0.03 |
| | Butyl parahydroxybenzoate | 0.03 | 0.03 | 0.03 |
| Solid or semi-solid hydrocarbon oil at ordinary temperature | White petrolatum | to a total of 100 | to a total of 100 | to a total of 100 |
| State of active ingredient present in composition | OCT | Dissolved | Dissolved | Dissolved |
| | BBP | Dispersed | Dispersed | Dispersed |
| Stability (50°C, 8 weeks) (Scale: 100 g) | OCT | 98.9% | 99.0% | 95.6% |
| | BBP | 101.4% | 100.2% | 100.6% |

As can be seen from Table 5, the residual ratio of each of the active ingredients of the compositions No. 5 and No. 6 according to the present invention was high even after the compositions were stored at an extreme temperature of 50°C for 8 weeks. On the other hand, the residual ratio of OCT of the comparative composition No. 7 was low.

From the results of Examples described above, it was revealed that the combined use of a low-polarity ester oil and a non-polar liquid solvent made it possible to provide a composition for skin that contains maxacalcitol and a betamethasone-based steroid as active ingredients, that is excellent in the stability of each of the active ingredients, and that shows transdermal absorbability of each of the active ingredients comparable to that of each of the single-drug products.

### [Example 4]

The efficacy of the composition No. 5 according to the present invention (ointment containing 0.0025% of OCT and 0.05% of BBP; hereinafter referred to as an ointment No. 5) for the treatment of patients with psoriasis vulgaris was compared to that of an ointment containing OCT alone (0.0025% OCT; hereinafter referred to as an OCT ointment) and that of an ointment containing BBP alone (0.05% BBP; hereinafter referred to as a BBP ointment).

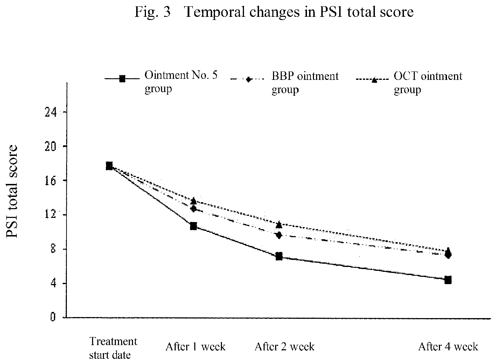

Table 6 shows the summary of a clinical trial protocol, and Fig. 3 shows temporal changes in PSI (Psoriasis Severity Index: an evaluation index that converts the severity of psoriatic lesion skin findings (erythema, infiltration/thickening, scales) into scores) acquired by 4-week clinical trials. It is to be noted that the reason why the OCT ointment was applied twice a day is that the approved administration of the OCT ointment is "usually twice a day". On the other hand, the approved administration of the BBP ointment is "once to several times a day", but the BBP ointment was administered once a day. This is because there is a report that there is not much difference in the efficacy of "very strong class" steroids including BBP between administration once a day and administration twice a day, and there is a report that the dose of a steroid should be as small as possible from the viewpoint of reducing its side effects. Similarly, the frequency of administration of the ointment No. 5 was also once a day.

**[Table 6]**

| Study design | Double-blind, parallel-group, comparative study | | |
|---|---|---|---|
| Target | Patients with psoriasis vulgaris | | |
| Key inclusion criteria | • Patients with a total BSA (a percentage of eruption area to body surface area) of the trunk, upper limbs, and lower limbs of less than 20% on the observation start date and treatment start date | | |
| | • Patients with an eruption assessed by PSI and satisfying the following criteria on the observation start date and treatment start date: | | |
| | 1) having an eruption area of 10 cm² or more; | | |
| | 2) eruptions being present in the trunk, upper limb or lower limb (except for an area beyond the wrist or ankle); and | | |
| | 3) having a total PSI score of 15 or more and a PSI score of each skin finding (erythema, infiltration/thickening, scales) of 4 or more | | |
| Key exclusion criteria | Patients with the following history: | | |
| | • Serious allergy (shock, anaphylactoid symptoms) | | |
| | • Allergy to vitamin D, vitamin D derivatives, or steroids | | |
| | • Dermal hypersensitivity to topically-administered drugs (contact dermatitis etc.) | | |
| Test schedule and test method | Observation period (2 weeks) | Treatment period (4 weeks) | |
| | Placebo Administration: twice/day | Ointment No. 5 (OCT 25 µg/g, BBP 500 µg/g) | |
| | | Morning: placebo Evening: Ointment No. 5 | |
| | | OCT ointment (OCT 25 µg/g) | |
| | | Morning: OCT ointment Evening: OCT ointment | |
| | | BBP ointment (BBP 500 µg/g) | |
| | | Morning: placebo Evening: BBP ointment | |

| | After the 2-week observation period, each of the test drugs is applied up to 10 g per day for 4 weeks to all the eruptions of psoriasis vulgaris in the trunk, upper limbs, and lower limbs (except for eruptions in the head and neck area (hair-covered scalp, face, and neck)). | | |
|---|---|---|---|
| Number of cases entered | Test drug | Number of cases entered | Total |
| | Ointment No. 5 | 166 cases | 475 cases |
| | OCT ointment | 156 cases | |
| | BBP ointment | 153 cases | |

As can be seen from Fig. 3, the composition according to the present invention (ointment No. 5) produced a larger improvement than both the single-drug products (BBP ointment and OCT ointment) after a lapse of 1 week from the start of administration, and showed a significantly higher efficacy than both the single-drug products in terms of the PSI total score after 4 weeks. Further, major adverse events that occurred in the ointment No. 5 group were nasopharyngitis and reduction in blood cortisol. However, both the adverse events were mild, and there was not much difference in adverse event rates between the composition according to the present invention and both the single-drug products, from which the safety of the composition according to the present invention was considered to be comparable to that of both the single-drug products.

### INDUSTRIAL APPLICABILITY

The composition for skin according to the present invention can keep both maxacalcitol and a betamethasone-based steroid, which are different in chemical properties, stable while achieving appropriate transdermal absorbabilities of both the active ingredients. This makes it possible to achieve excellent drug efficacy without using two single-drug products containing each of the active ingredients alone.

## Claims

1. A non-aqueous composition for skin, comprising:
(a) maxacalcitol;
(b) at least one corticosteroid selected from the group consisting of betamethasone and esters thereof:
(c) at least one low-polarity ester oil having an IOB value of 0.07 or more but less than 0.20; and
(d) at least one non-polar liquid solvent.

2. The composition according to claim 1, wherein a content of the low-polarity ester oil (c) is 1.5 to 4 wt%, a content of the non-polar liquid solvent (d) is 2 to 15 wt%, and the content of the (d) is higher than the content of the (c).

3. The composition according to claim 1 or 2, wherein the low-polarity ester oil (c) is selected from the group consisting of octyldodecyl myristate, isostearyl palmitate, hexyldecyl isostearate, oleyl oleate, isocetyl myristate, stearyl stearate, decyl oleate, ethylhexyl stearate, cetyl caprate, octyl palmitate, cetyl 2-ethylhexanoate, isopropyl isostearate, hexyl laurate, isopropyl palmitate, isopropyl linoleate, and isopropyl myristate.

4. The composition according to any one of claims 1 to 3, wherein the low-polarity ester oil (c) is selected from the group consisting of octyldodecyl myristate, isostearyl palmitate, hexyldecyl isostearate, oleyl oleate, isocetyl myristate, and stearyl stearate.

5. The composition according to any one of claims 1 to 4, wherein the non-polar liquid solvent (d) is selected from the group consisting of liquid paraffin and squalane.

6. The composition according to any one of claims 1 to 5, further comprising 80 wt% or more of a hydrocarbon oil that is solid or semi-solid at 25°C.

7. The composition according to claim 6, wherein the hydrocarbon oil is petrolatum.

8. The composition according to claim 1, wherein
the component (b) is betamethasone butyrate propionate,
the component (c) is selected from octyldodecyl myristate and oleyl oleate, and the content thereof is 1.5 to 4 wt%,
the component (d) is selected from liquid paraffin and squalane, and the content thereof is 2 to 15 wt%,
80 wt% or more of petrolatum is contained, and
a weight ratio between the component (c) and the component (d) is 1 : 1.3 to 4.5

9. The composition according to claim 1, wherein
the component (b) is betamethasone butyrate propionate,
the component (c) is 1.5 to 2.5 wt% of octyldodecyl myristate,
the component (d) is 2.5 to 5 wt% of liquid paraffin,
90 wt% or more of petrolatum is contained, and
a weight ratio between the components (c) and (d) is 1 : 1.3 to 2.
